# EUROPÄISCHE PATENTANMELDUNG

(11) **EP 4 533 962 A1**
(43) Veröffentlichungstag der Anmeldung: **09.04.2025**
(21) Anmeldenummer: 24201140.1
(22) Anmeldetag: 18.09.2024
(51) Int. Cl.: A23L 33/105, A23L 33/15, A23L 33/16, A23L 33/185, A61K 8/65, A61K 36/185, A61K 38/39, A61P 17/00, A61Q 19/00, A61Q 19/08

(54) **ZUSAMMENSETZUNG ZUR VERWENDUNG ALS NAHRUNGSERGÄNZUNGSMITTEL ZUR PRODUKTION VON FÜR DAS HAUTBILD RELEVANTEN KURZKETTIGEN PEPTIDEN IM VERDAUUNGSTRAKT**

(30) Priorität: 04.10.2023 DE 102023209700
(71) Anmelder: Quiris Healthcare GmbH & Co. KG, 33334 Gütersloh (DE)
(72) Erfinder: Lange, Peer, 33334 Gütersloh (DE)
(74) Vertreter: BRP Renaud & Partner mbB Rechtsanwälte Patentanwälte Steuerberater

(57) **Zusammenfassung**

Die vorliegende Erfindung betrifft eine Zusammensetzung zur Verwendung als Nahrungsergänzungsmittel zur Produktion von für das Hautbild relevanten kurzkettigen Peptiden im Verdauungstrakt, zumindest enthaltend die Komponenten Kollagen-Peptide bovinen Ursprungs mit einer Molekularmasse von durchschnittlich 2000 Da, die sich durch eine hohe Sequenzhomologie mit menschlichem Kollagen Typ I α-1, Typ I α-2 und Typ III α-1 mit einer Gesamt-Sequenzabdeckung von größer als 90 % für humanes Kollagen Typ I α-1 und α-2 auszeichnen und einem wässrigen Extrakt aus Acerola-Frucht (Malpighia punicifolia), der sich neben dem standardisierten Vitamin C-Gehalt durch das Vorhandensein löslicher Ballaststoffe mit einem hohen Uronsäuregehalt auszeichnet. Die Erfindung eignet sich insbesondere als Nahrungsergänzungsmittel zur Erhaltung der Hautgesundheit.

## Beschreibung

Die vorliegende Erfindung betrifft eine Zusammensetzung zur Verwendung als Nahrungsergänzungsmittel zur Produktion von für das Hautbild relevanten kurzkettigen Peptiden im Verdauungstrakt, zumindest enthaltend bovine Kollagen-Peptide mit einer Molekularmasse von durchschnittlich 2000 Da und einem Extrakt aus der Acerola-Frucht (Malpighia punicifolia).

Mit zunehmendem Lebensalter ist die menschliche Haut sichtbaren Zeichen der Hautalterung unterworfen, die in Abhängigkeit der genetischen Disposition und von Lebensstilfaktoren unterschiedlich früh und unterschiedlich stark ausgeprägt auftreten können. Im Gesicht sind diese Zeichen der Zeit besonders offensichtlich. Seit Urzeiten ist der Einsatz kosmetischer Mittel zu dekorativen Zwecken belegt, wobei besonders in den letzten Jahrzehnten den hautpflegenden Eigenschaften topischer Zubereitungen eine immer größere Bedeutung zugeschrieben wird. Aufgrund der starken Barriereeigenschaften der Basallamina in der menschlichen Haut kann der Großteil der, von außen applizierten Substanzen, Wirkungen jedoch allenfalls in der äußersten Hautschicht, der Epidermis, entfalten.

Aus diesem Grund wurden in den vergangenen Jahren immer mehr sogenannte Nutraceuticals entwickelt. Dabei handelt es sich meist um Nahrungsergänzungsmittel, die für die Gesundheit der Haut relevante Nährstoffe enthalten. Beispielsweise offenbart die DE 10 2016 107 508 A1 eine orale Zusammensetzung enthaltend Kollagenhydrolysat und Vitamin C zur Herstellung eines funktionellen Lebensmittels oder eines Nahrungsergänzungsmittels zur Förderung des Erhalts gesunder Haut.

Neben Mikronährstoffen wie Biotin, Zink, Vitamin C, Jod, Kupfer, Niacin, Riboflavin oder Vitamin A, denen von der europäischen Lebensmittelbehörde (EFSA) hautrelevante Effekte in Form so genannter Health Claims bescheinigt wurden, sind außerdem Pflanzenextrakte oder selektierte Bestandteile der extrazellulären Matrix tierischer Gewebe beliebte Inhaltsstoffe derartiger Produkte, wobei der Wirkungsmechanismus oft unklar bleibt.

Ein generell in Nahrungsergänzungsmitteln häufig eingesetzter Pflanzenextrakt wird aus der Acerola-Frucht gewonnen. Sie wird für ihren sehr hohen Vitamin C-Gehalt geschätzt und entsprechende Extrakte zur nutritiven Anwendung sind zumeist (nur) auf diesen einen Inhaltsstoff standardisiert. Allenfalls antioxidative Begleitstoffe wie Polyphenole werden hinsichtlich ihrer ernährungsphysiologischen Wirkungen in der Literatur zuweilen thematisiert.

Von den oben genannten Bestandteilen der extrazellulären Matrix erfreut sich insbesondere die Anwendung von Kollagen-Peptiden großer Beliebtheit. Kollagen ist das häufigste Strukturprotein im menschlichen Körper. Gleiches gilt für andere Wirbeltiere, sodass kollagenhaltige Bestandteile als genusstaugliches Beiprodukt bei der Fleisch- und Fischproduktion anfallen. Während die Verarbeitung dieser Bestandteile zu Gelatine eine lange Tradition hat, hat die Herstellung von Kollagen-Peptiden in den letzten Jahren stark an Bedeutung gewonnen.

Kollagen-Peptide unterscheiden sich von Gelatine sowohl in ihren galenischen Eigenschaften (z.B. Gelierfähigkeit) als auch in ihrer Bioverfügbarkeit. Welche Eigenschaften ihre Wirkungen auf das Hautbild maßgeblich beeinflussen, ist aktuell Gegenstand wissenschaftlicher Forschung. Die ursprüngliche These, dass sie dem Körper ausschließlich die für die Proteinsynthese notwendigen Aminosäuren zur Verfügung stellen ("Bausteinfunktion"), kann als widerlegt angesehen werden. Stattdessen gibt es Hinweise, dass sowohl die Auswahl der tierischen Quelle (bovin, porcin, marin) als auch die Prozessführung im Hydrolyseverfahren (beispielsweise die eingesetzten Proteasen) die Beschaffenheit und Größe und damit auch die (Signal-)Wirkung der Kollagen-Peptide beeinflussen.

Während man in der Vergangenheit vor allem Hydroxyprolin-haltigen Peptiden eine große Bedeutung beimaß, weil diese besonders kollagentypisch sind, legen jüngere Forschungsarbeiten nahe, dass auch andere Peptid-Bruchstücke eine ernährungsphysiologische Bedeutung haben. Die Arbeiten der Forschungsgruppe um Zhao Deng et al. haben jüngst vorgestellt, dass sich Peptide, die die Sequenz PGA (Prolin-Glycin-Alanin) enthalten, durch eine starke anti-inflammatorische Aktivität auszeichnen. Vor dem Hintergrund, dass Entzündungsprozesse auch zur Hautalterung beitragen, ist diese Erkenntnis auch für die Erhaltung der Hautgesundheit von großem Interesse.

Nachteilig an den oben genannten Forschungsergebnissen ist, dass diese auf synthetisch hergestellten Peptiden bzw. Peptid-Bruchstücken basiert. Es besteht daher der Bedarf, den Wirkungsmechanismus solcher Zubereitungen näher zu untersuchen, damit auf dieser Basis bestehende Zubereitungen in ihrer Wirkung verbessert werden können.

Aufgabe der vorliegenden Erfindung ist es, eine naturbasierte Quelle derartiger Peptide zu identifizieren, die zu nutritiven Zwecken genutzt werden kann. Aufgabe der vorliegenden Erfindung ist es auch, diese Peptide in einer Zusammensetzung bereitzustellen, welche gegenüber bekannten Zusammensetzungen zur ernährungsphysiologischen Beeinflussung des Hautbilds verbesserte Eigenschaften aufweist.

Gelöst wird die Aufgabe durch eine Zusammensetzung zur Verwendung als Nahrungsergänzungsmittel gemäß Anspruch 1. Weitere bevorzugte Ausführungsformen ergeben sich aus den abhängigen Ansprüchen.

Überraschend ist es gelungen, durch die Kombination von Kollagen-Peptiden mit einem, bestimmte Polysaccharide enthaltenden Acerola-Frucht-Extrakt, die beim menschlichen Verdau entstehenden Peptid-Bruchstücke derart zu beeinflussen, dass bevorzugt solche entstehen, die die physiologisch relevante Sequenz PGA enthalten.

Unter dem Begriff "Extrakt" werden im Sinne dieser Erfindung konzentrierte, gegebenenfalls auf einen bestimmten Gehalt an Inhaltsstoffen eingestellte, Auszüge aus Früchten oder anderen Bestandteilen von Pflanzen oder aus tierischen Bestandteilen von flüssiger, zähflüssiger, öliger oder trockener Beschaffenheit verstanden. Extrakte auf pflanzlicher Basis können beispielsweise unter Verwendung von Alkohol oder Wasser gewonnen werden. Unter dem Begriff "Extrakt" werden im Sinne dieser Erfindung ebenfalls gereinigte Extrakte oder Extraktfraktionen verstanden. Unter dem Begriff "Extrakt aus" wird im Sinne dieser Erfindung verstanden, dass der Extrakt aus der entsprechenden Pflanze oder Pflanzenteilen bzw. tierischen Bestandteilen erhältlich oder hergestellt ist.

Erfindungsgemäß werden bovine Kollagen-Peptide mit einem Molekulargewicht von durchschnittlich 2000 Dalton eingesetzt, die, vorzugsweise durch Hydrolyse von Gelatine aus, vorzugsweise Rinderhaut, unter Einwirkung verschiedener Proteasen hergestellt werden. Die Charakterisierung des Rohstoffes mittels LC-MS/MS-Messungen unter Verwendung einer Umkehrphasen-nano LC-Säule zeigt, dass die Peptide eine Kettenlänge von bevorzugt weniger als 26 Peptiden aufweisen.

Nach Analyse der Primärstruktur der Kollagenbestandteile und Vergleich mit einschlägigen Proteindatenbanken kann eine hohe, Sequenzhomologie mit dem humanen Kollagen Typ I α-1 von 31 %, dem humanen Kollagen Typ I α-2 von 18 % und dem humanen Kollagen Typ III α-1 von 13 % festgestellt werden, die höher liegt als die Sequenzabdeckung von Kollagen-Peptiden anderer Herkunft mit 4 bis 20 %, 7 bis 16 % und 6 bis 11 %. Die Gesamt-Sequenzabdeckung für humanes Kollagen Typ I α-1 liegt bei über 94 %, die für humanes Kollagen Typ I α-2 bei über 90 %. Wegen des Gehalts an ernährungsphysiologisch wertvollem Protein und der Abwesenheit zugesetzter Konservierungsstoffe stellen die erfindungsgemäßen Kollagenbestandteile ein hochwertiges Nahrungsergänzungsmittel dar.

Der verwendete Acerola-Frucht-Extrakt ist nicht nur auf einen festen Gehalt von Vitamin C standardisiert, sondern zeichnet sich zudem durch einen nennenswerten Gehalt an hochmolekularen löslichen Ballaststoffen aus, wie sich mittels enzymatischer Gravimetrie zeigen lässt. Durch spektrophotometrische Methoden lässt sich weiterhin ein ähnlicher hoher Gehalt an Uronsäuren feststellen. Eigene Messungen belegen einen Ballaststoffgehalt von mindestens 2,1 % (Gravimetrie) mit einem hohen Uronsäuregehalt von mindestens 1,6 %. (Spektrometrie) für den spezifischen hier eingesetzten Acerola-Frucht-Extrakt. Uronsäuren sind Monosaccharide, deren primäre Alkoholfunktionen (CH₂OH-Gruppe, C6) zu Carbonsäurefunktionen (COOH-Gruppe) oxidiert sind. Das Molekül besitzt also sowohl eine Carbonyl-(Aldehyd) an C1 als auch eine Carboxylfunktion an C6. Dies lässt den Schluss zu, dass es sich bei den genannten Ballaststoffen um Pektin-Derivate handeln könnte. Es ist bekannt, dass hochmolekulare Bestandteile wie Ballaststoffe die Resorption anderer Nahrungsbestandteile im menschlichen Darm beeinflussen können.

Erfindungsgemäß weist der wässrige Extrakt aus der Acerola-Frucht den standardisierten Vitamin C-Gehalt, einen Gehalt, bevorzugt von 0,2 Gew.-% bis 10,0 Gew.-%, besonders bevorzugt von 1,0 Gew.-% bis 5,0 Gew.-% an, bevorzugt hochmolekularen löslichen Ballaststoffen, sowie einen Gehalt, bevorzugt von 0.1 Gew.-% bis 5,0 Gew.-%, besonders bevorzugt von 0,5 Gew.-% bis 2,5 Gew.-% an Uronsäuren auf (die typischen/bevorzugten Säuren sollten zumindest beispielhaft genannt werden, so dass die Erfindung so deutlich und vollständig offenbart ist, dass ein Fachmann sie ausführen kann). Die Uronsäuren sind D-Glucuronsäure, D-Galacturonsäure und D-Mannuronsäure. Es dominiert die D-Galacturonsäure. Daher gehen die Erfinder davon aus, dass es sich bei den Ballaststoffen um Pektine handelt. Pektine sind Polyuronide, die im Wesentlichen aus α-1,4-glycosidisch verknüpften D-Galacturonsäure-Einheiten bestehen.

Da für die ernährungsphysiologischen Wirkungen in der Haut maßgeblich ist, in welcher Form oral zugeführte Inhaltsstoffe dort ankommen, wurde für ein besseres Verständnis der Verdauungsprozesse das Modell "TinyTIM" der Firma Triskelion verwendet. Hierbei handelt es sich um ein validiertes in vitro-System, das die Prozesse im menschlichen Verdauungstrakt nachahmt. Dazu werden verschiedene Kompartimente (gastrisch, intestinal) benutzt, die durch ein peristaltisches Ventil verknüpft sind. Im Rahmen einer dynamischen Prozessführung werden die Bedingungen im menschlichen Gastrointestinaltrakt mittels u.a. Temperatur, pH-Wert, Elektrolyten, Verdauungsenzyme, Mechanik simuliert.

Hierbei zeigte sich überraschend, dass nach dem Verdau einer Kombination aus Kollagenpeptiden und Acerola-Extrakt diverse kurzkettige Peptid-Bruchstücke deutlich häufiger nachzuweisen waren als nach dem Verdau von Gelatine.

Überraschenderweise konnte gezeigt werden, dass viele dieser Peptide, die für eine entzündungshemmende Wirkkomponente relevante Aminosäuresequenz GPA enthalten. Unter den 10 am häufigsten auftretenden Peptid-Bruchstücken fanden sich die vier Peptide: GPAGPAGPR, GPAGV, GRpGApGPA und VGPA (Einbuchstabencode für Aminosäuren; p = Hydroxyprolin).

In vorteilhafterweise beeinflusst die erfindungsgemäße Kombination von Kollagen-Peptiden und Acerolaextrakt, jeweils mit den beschriebenen Eigenschaften, den Verdauungsprozess im menschlichen Körper derart, dass bevorzugt Peptid-Bruchstücke mit der gewünschten Aminosäuresequenz entstehen.

Die eingangs formulierte Aufgabe wird demnach gelöst von einer Zusammensetzung gemäß Anspruch 1 und deren Verwendung als Nahrungsergänzungsmittel. Weitere bevorzugte Ausführungsformen ergeben sich aus den abhängigen Ansprüchen.

In anderen Worten wird die Aufgabe gelöst von einer Zusammensetzung zur Verwendung als Nahrungsergänzungsmittel zur Produktion von für das Hautbild relevanten kurzkettigen Peptiden im Verdauungstrakt zumindest enthaltend die Komponenten
a) Kollagen-Peptide bovinen Ursprungs mit einer Molekularmasse von durchschnittlich 2000 Da und
b) wässriger Extrakt aus Acerolafrucht mit einem standardisierten Vitamin C-Gehalt, einem Gehalt von 0,2 Gew.-% bis 10,0 Gew.-% an hochmolekularen löslichen Ballaststoffen und einem Gehalt von 0,1 Gew.-% bis 5,0 Gew.-% an Uronsäuren.

In einer bevorzugten Ausführungsform umfasst die Zusammensetzung, jeweils bezogen auf das Gesamtgewicht derselben weiterhin mindestens die Komponenten
a) Vitamin E im Bereich von 1 × 10⁻⁴ Gew.-% bis 4 Gew.-%, vorzugsweise im Bereich von 4 x 10⁻⁴ Gew.-% bis 1 Gew.-%, besonders bevorzugt im Bereich von 1 × 10⁻³ Gew.-% bis 1 × 10⁻¹ Gew.-%,
b) Biotin im Bereich von 4 × 10⁻⁶ Gew.-% bis 0,02 Gew.-%, vorzugsweise im Bereich von 1 × 10⁻⁵ Gew.-% bis 1 × 10⁻³ Gew.-%, besonders bevorzugt im Bereich von 1 × 10⁻⁴ Gew.-% bis 1× 10⁻³ Gew.-% und
c) Zinkcitrat im Bereich von 4 x 10⁻⁴ Gew.-% bis 0,08 Gew.-%, vorzugsweise im Bereich von 1 × 10⁻³ Gew.-% bis 1 × 10⁻¹ Gew.-%, besonders bevorzugt im Bereich von 1 × 10⁻² Gew.-% bis 1 × 10⁻¹ Gew.-%, wobei die Gewichtsgehalte der einzelnen Komponenten so gewählt sind, dass das Gesamtgewicht der Komponenten 100 Gew.-% nicht übersteigt.

Vorzugsweise sind in der Zusammensetzung a) Kollagen-Peptide und b) Extrakt aus Acerolafrucht im Gewichtsverhältnis 8:1 bis 2:1, höchst bevorzugt im Verhältnis 3,75:1 enthalten.

Neben den Komponenten a), b) und ggf. c) bis e) kann die Zusammensetzung in einer bevorzugten Ausführungsform weitere Mikronährstoffe und/oder Pflanzenextrakte sowie galenische Hilfsstoffe (Zucker, Süßungsmittel, Stabilisatoren, Säureregulatoren, Konservierungsmittel, Geschmackskorrigenzien) enthalten.

## Patentansprüche

1. Zusammensetzung zur Verwendung als Nahrungsergänzungsmittel zur Produktion von für das Hautbild relevanten kurzkettigen Peptiden im Verdauungstrakt zumindest enthaltend die Komponenten
a) Kollagen-Peptide bovinen Ursprungs mit einer Molekularmasse von durchschnittlich 2000 Da und
b) wässriger Extrakt aus Acerolafrucht mit einem standardisierten Vitamin C-Gehalt, einem Gehalt von 0,2 Gew.-% bis 10,0 Gew.-% an hochmolekularen löslichen Ballaststoffen und einem Gehalt von 0.1 Gew.-% bis 5,0 Gew.-% an Uronsäuren.

2. Zusammensetzung nach Anspruch 1, **dadurch gekennzeichnet, dass** die Kollagen-Peptide eine Kettenlänge von weniger als 26 Peptiden aufweisen.

3. Zusammensetzung nach Anspruch 2, **dadurch gekennzeichnet, dass** die Kollagen-Peptide eine hohe Sequenzhomologie mit dem humanen Kollagen in der Haut von Typ I α-1, Kollagen Typ I α-2 und Kollagen Typ III α-1 aufweisen. Die Gesamt-Sequenzabdeckung des humanes Kollagen Typ I α-1 liegt bei über 94 %, für humanes Kollagen Typ I α-2 bei über 90 %.

4. Zusammensetzung nach Anspruch 1, 2 oder 3, **dadurch gekennzeichnet, dass** diese jeweils bezogen auf das Gesamtgewicht derselben weiterhin mindestens die Komponenten
c) Vitamin E im Bereich von 1 × 10⁻⁴ Gew.-% bis 4 Gew.-%,
d) Biotin im Bereich von 4 × 10⁻⁶ Gew.-% bis 0,02 Gew.-% und
e) Zinkcitrat im Bereich von 4 × 10⁻⁴ Gew.-% bis 0,08 Gew.-% enthält,
wobei die Gewichtsgehalte der einzelnen Komponenten so gewählt sind, dass das Gesamtgewicht der Komponenten 100 Gew.-% nicht übersteigt.

5. Zusammensetzung nach einem der vorgenannten Ansprüche, **dadurch gekennzeichnet, dass** a) und b) im Gewichtsverhältnis von 8:1 bis 2:1 enthalten sind.

6. Zusammensetzung nach einem der vorstehenden Ansprüche, **dadurch gekennzeichnet, dass** diese weitere Mikronährstoffe und/oder Pflanzenextrakte sowie galenische Hilfsstoffe enthält.

7. Zusammensetzung gemäß einem der vorgenannten Ansprüche zur Verwendung als Nahrungsergänzungsmittel zur Erhaltung der Hautgesundheit.

8. Zusammensetzung gemäß einem der vorgenannten Ansprüche in der Darreichungsform Trinkampulle.
